Europäisches Patentamt

**European Patent Office** (11) Publication number: **0 218 359**

Office européen des brevets A1

## (12) EUROPEAN PATENT APPLICATION

(21) Application number: **86306729.4**

(51) Int. Cl.⁴: **C 07 C 7/11,** C 10 G 5/04

(22) Date of filing: **01.09.86**

(30) Priority: **04.10.85 US 784566**
**13.12.85 US 808463**
**13.02.86 US 828996**

(43) Date of publication of application: **15.04.87**
**Bulletin 87/16**

(84) Designated Contracting States: **AT BE CH DE FR GB IT LI LU NL SE**

(71) Applicant: **EL PASO HYDROCARBONS COMPANY, 620 N. Grant Street, Odessa, TX 79761 (US)**

(72) Inventor: **Mehra, Yuv R., 2708 Halifax Ave., Odessa Texas 79762 (US)**

(74) Representative: **Jukes, Herbert Lewis, Swann, Elt & Company 31 Beaumont Street, Oxford OX1 2NP (GB)**

(54) Conditioning natural gas streams with preferential physical solvents.

(57) A process is disclosed in which a preferential physical solvent selectively extracts and strips desirable $C_2+$ hydrocarbons from a raw hydrocarbon gas stream which is fed into the midsection of an Extractor-Stripper (ES) column, the rich solvent produced from the bottoms thereof being regenerated in a distillation column which produces a hydrocarbon gas liquids stream as product and a lean physical solvent stream for recycling to the top of the ES column. Suitable preferential physical solvents include $C_8$–$C_{10}$ aromatic compounds having methyl, ethyl, or propyl aliphatic groups, including mesitylene, n-propyl benzene, n-butyl benzene, o-xylene, m-xylene, p-xylene, and mixtures thereof, and certain reformate streams rich in mixed xylenes and other $C_8$–$C_{10}$ aromatics. By substituting this solvent for the lean oil solvent in a conventional absorption-type plant for treating natural gas, the plant can produce a residue gas stream meeting specifications for methane content and a hydrocarbon liquids product having a selected composition.

EPH-86-7

## CONDITIONING NATURAL GAS STREAMS
## WITH PREFERENTIAL PHYSICAL SOLVENTS

This invention relates to contacting a hydrocarbon-containing gas stream with a preferential physical solvent. It more specifically relates to separating and recovering ethane and higher boiling hydrocarbons from a hydrocarbon-containing gas stream and especially relates to simplification of the Mehra Process by elimination of the flashing step. It additionally relates to rejuvenation of conventional lean oil absorption plants which recover hydrocarbons from a natural gas stream. It further relates to specific preferential physical solvents for extractive stripping of a hydrocarbon gas stream.

Due to the market demand for lighter hydrocarbons and the lack of selectivity of lean oils for such components, the absorption process has been gradually replaced by improved processes known as refrigerated oil absorption, simple refrigeration, cascaded refrigeration, Joule-Thompson, and cryogenic expander processes. The extractive flashing (EF) version of the Mehra Process is disclosed in U.S. Patents 4,421,535; 4,511,381; 4,526,594; 4,578,094; and 4,601,738. This EF version utilizes preferential physical solvents for extracting selected amounts of $C_2$-$C_4$ hydrocarbons and all $C_5$+ hydrocarbons from a natural gas by using the steps of flashing, recycling of $C_1$-rich flashed gases, and compression, cooling, and condensing of $C_1$-lean flashed gases, followed by demethanizing the condensate to produce a natural gas liquids (NGL) product and an overhead gas stream which is recycled to the extraction step. Typical recoveries for these processes are compared in Table I.

## TABLE I

### COMPARISON OF TYPICAL LIQUID RECOVERIES

| EXTRACTION | ETHANE (%) | PROPANE (%) | BUTANES (%) | GASOLINE (%) |
|---|---|---|---|---|
| ABSORPTION | 5 | 40 | 75 | 87 |
| REFRIGERATED ABSORPTION | 15 | 75 | 90 | 95 |
| SIMPLE REFRIGERATION | 25 | 55 | 93 | 97 |
| CASCADED REFRIGERATION | 70 | 85 | 95 | 100 |
| JOULE-THOMPSON EXPANSION | 70 | 90 | 97 | 100 |
| TURBO-EXPANDER | 85 | 97 | 100 | 100 |
| MEHRA PROCESS (EF) | 2-90 | 2-99 | 2-100 | 100 |

-3-

-4-

In summary, the oil absorption, refrigerated oil absorption, simple refrigeration, and cascaded refrigeration processes operate at the pipeline pressures, generally without letting down the gas pressure, but the recovery of desirable liquids (ethane plus heavier components) is poor, with the exception of the cascaded refrigeration process which has extremely high operating costs but achieves good ethane and propane recoveries. The Joule-Thompson and the cryogenic turbo-expander processes achieve high ethane recoveries by letting down the pressure of the entire inlet gas, which is primarily methane (typically 80-85%), but recompression of most of the inlet gas is quite expensive.

In all of the above processes, except the Mehra Process, the ethane-plus-heavier components are recovered in a specific configuration determined by their composition in the raw hydrocarbon gas stream and equilibrium at the key operating conditions of pressure and temperature within the process.

Under poor economic conditions when the ethane price as petrochemical feedstock is less than its equivalent fuel price and when the propane price for feedstock usage is attractive, the operator of a hydrocarbon gas liquids extraction plant is limited as to operating choice because he is unable to minimize ethane recovery and maximize propane recovery in response to market conditions.

The simple refrigeration process, which typically recovers 55% of the propane, also typically requires the recovery of 25% of the ethane. In order to boost propane recovery to the 95+% level, cascaded refrigeration, Joule-Thompson, or cryogenic turbo-expander processes have to be used while simultaneously boosting the ethane recovery to 70+% at a considerably larger capital investment.

In carrying out the extraction of desired hydrocarbons according to the extractive flashing embodiment of the Mehra Process, the energy consumption is lower than for conventional state-of-the-art processes, but several steps are required that increases the complexity and overall capital requirements of such a plant. There is consequently a need for simplification of the Mehra Process in order to reduce its capital investment requirement.

The Mehra Process, in its preferred mode, also utilizes a mixture of dialkyl ethers of polyalkylene glycol, having a molecular weight of 146 to 476 and containing 3-10 ethylene units, for example. While such compounds are satisfactory for the extractive flashing embodiment of the Mehra Process, they are subject to possible further polymerization and/or thermal degradation if cyclically flowing through a unit operation requiring heating, such as distillation, at process temperatures used for separation of mixtures into useful fractions or components. There is, therefore, a need for other solvents that are not subject to these limitations.

U.S. Patent Nos. 2,290,957; 2,433,286; 2,455,803; 2,559,519; 2,570,066; 3,236,029; and 3,445,537 disclose multiple extractions or extractive distillations representing process simplifications.

-6-

It is accordingly an object of this invention to provide a method for simplifying the arrangement of equipment and for minimizing the capital investment needed for the EF version of the Mehra Process.

It is an additional object to provide a process for selectively extracting desired hydrocarbon components from a hydrocarbon gas stream with a stream of a preferential physical solvent to produce a solvent rich in $C_1+$ hydrocarbons.

It is a further object to provide a process for selectively rejecting undesired hydrocarbons from the stream of $C_1+$-rich solvent.

It is another object to provide certain preferential physical solvents that are not susceptible to thermally induced degradation or polymerization at process temperatures used in extraction-stripping and fractionation of hydrocarbon gases.

It is also an object to provide a process for selectively recovering 2-95% of the ethane in an existing gas absorber plant without having to make substantial changes in its apparatus and/or piping.

It is yet another object of this invention to provide a process for recovering up to 100% of propane in an existing gas absorber plant without having to make substantial changes in its apparatus and/or piping.

In accordance with these objects and the principles of this invention, an improved Mehra Process is herein provided in which a raw hydrocarbon gas stream is contacted with a preferential physical solvent in an Extractor-Stripper (ES) column, containing a stripping section and a superimposed extracting section, or in separate extracting and stripping columns which are sequentially operated as to all streams. However, the process is generally discussed hereinafter for illustrative purposes with respect to ES columns containing both sections.

The gas enters the ES column below the extraction section and flows upwardly where it contacts lean preferential physical solvent which, after entering the extraction section at the top of the column, flows downwardly in a countercurrent manner. The contact takes place over mass transfer surfaces, such as packing or distillation trays. The enriched solvent leaving the bottom of the extraction section is rich in $C_1$ and heavier hydrocarbons.

This $C_1$-rich solvent enters the stripping section of the ES column and flows downwardly, where it comes in contact with the upward-flowing rejected hydrocarbons created by the reboiler at the bottom of the ES column. The rejected hydrocarbons consist primarily of undesired hydrocarbons, such as $C_1$ if the desired objective is recovery of $C_2+$ hydrocarbons, or $C_1$ and $C_2$ if the desired objective is the recovery of $C_3+$ hydrocarbons, and so forth, depending upon the desired recovery objectives, plus some desirable hydrocarbons.

The selective recovery of $C_2+$ components is controlled by variations in flow rates of the lean preferential physical solvent within the extraction section of the ES column. Additional selectivity in this invention is provided by adding a reboiler and a stripping section in the extraction column, thereby gaining one more degree of freedom. This additional degree of freedom is effectively utilized by appropriately selecting the reboiling temperature in order to produce the rich solvent stream, consisting essentially of only the economically desired hydrocarbons, and rejecting the economically undesired hydrocarbons. Instead of a reboiler, a stripping stream of inert gas may be utilized for selective rejection of the undesirable hydrocarbons.

The rejected hydrocarbons, flowing upwardly as vapor through the stripping section of the ES column, contain undesirable hydrocarbons and some of the desirable hydrocarbons which are recovered preferentially by the downwardly flowing solvent, according to mass transfer principles developed for distillation as the solvent flows over the packing or trays of the ES column. The remaining undesirable hydrocarbons leave the stripping section of the ES column, join the incoming raw gas stream, and form a mixture which flows upwardly in the extraction section of the ES column, where lean physical solvent preferentially recovers any contained desired hydrocarbons.

The rich solvent leaving the bottom of the ES column is let down in pressure to a pressure level consistent with the operation of the gas liquids product column. This pressure level also obviates the need for a downstream compressor or pump. The rich solvent may be economically heated before entering the final liquids product column in order to lower the reboiler heat load and improve separation of hydrocarbons from the physical solvent.

The final liquids product column is a typical fractionation-type column in which the selectively extracted hydrocarbons are separated from the preferential physical solvent. The desired hydrocarbons are recovered from the top of the liquids product column while the hot, lean solvent leaves the bottom of the liquids product column. The temperature at the bottom of the liquids product column is selected to ensure the recovery of all desirable hydrocarbons and is no higher than the boiling point of the physical solvent at the operating pressure. In order to minimize the loss of the physical solvent with the hydrocarbon gas liquids product, the column overhead is refluxed with the condensed hydrocarbons.

In order to minimize the energy consumption within the process of this invention, the hot, lean physical solvent, leaving from the bottom of the liquids product column, is effectively utilized for heating the rich solvent feed to the liquids product column and for reboiling the ES column before returning to the top of the extraction section of the ES column as cool, lean preferential physical solvent.

It is important to note that in the process described so far, there is no external recycle of any streams. Furthermore, this process has been essentially reduced to a two-step process. Thus, the capital requirements of this process are considerably reduced over the previous versions of the Mehra process, as described in U. S. Patents Nos. 4,421,535; 4,511,381; 4,525,594; 4,578,094; and 4,601,738.

This two-step version of the Mehra Process necessitates that the rich solvent, leaving the bottom of the ES column, contain only the specified amounts of the undesirable lighter components, such as $C_1$ in $C_2$+ products, in order to meet the liquids product specification typically set if the hydrocarbon gas is natural gas.

In the extractive flashing version of the Mehra Process, this specification has been effectively achieved by selecting the operating pressure and temperature at the bottom of the demethanizing or stripping column. Because such a purity requirement has been combined with selectivity in the ES column of this invention, wherein the selection capability of operating pressure is unavailable because it is generally determined by the delivery pressure of the residue gas, only temperature flexibility at the bottom of the ES column is available for meeting the required specification of undesirable components because the other flexibility of flow rate of preferential physical solvent to the ES column is effectively utilized in meeting the selective recovery levels of desired hydrocarbon components of the raw gas stream.

In summary, the extraction section of the ES column is used for extracting desired hydrocarbon components from the hydrocarbon gas stream to form an extracted liquid (in solution in the solvent), and the stripping section of the ES column is used for rejecting the undesired components from the extracted liquid. Under certain operating conditions and for certain liquids product specifications, it may become necessary to operate the ES column bottoms at temperatures high enough to become relatively energy inefficient. It may thus be economically viable and preferred to allow additional quantities of undesirable hydrocarbons to be present in the rich solvent leaving the bottom of the ES column. These contained undesirable hydrocarbons would lower the temperature at the bottom of the ES column and thus allow for an effective energy recovery loop composed of rich/lean solvent streams.

The contained undesirable hydrocarbons can be effectively removed by providing a single-stage intermediate flashing vessel, operating at a pressure consistent with the operating pressure of the liquids product column. The separated vapor stream contains all of the undesirable components along with some desirable hydrocarbons. This stream is compressed to the pressure levels consistent with the operating pressure of the ES column. The compressed vapors are recycled to the ES column, at approximately the inlet height for the hydrocarbon gas stream, for additional recovery of desirable hydrocarbons.

The operating pressure and the temperature conditions of the ES column can vary from 1,500 to 9,100 Kpa absolute and -18°C to 320°C, respectively, 2,200 to 4,200 Kpa (abs.) being a preferred operating pressure range for the ES column. Similarly, the operating pressure and temperature at the bottom of the liquids product column can vary from 200 to 3,600 Kpa (abs.) and 66°C to 430°C, respectively, 1,100 to 2,500 Kpa (abs.) being a preferred range of operation for the liquids product column. The operating temperature at the top of the liquids product column is determined by the equilibrium conditions for the desired composition of the hydrocarbon liquid product that are consistent with the market conditions and the operating pressure of the column. The operating pressure of the liquids product column is selected such that the desired hydrocarbon can be condensed by reasonably available cooling media such as ambient air, cooling water, or warm level refrigerant. The bottoms temperature of the liquids product column is preferred to be equivalent to the boiling point of the physical solvent at the operating pressure in order to ensure maximum recovery of desirable hydrocarbons.

An interesting phenomenon associated with a preferential physical solvent, as defined by relative volatility and loading factor or by preference factor, is that the relative volatility or selectivity of each hydrocarbon being absorbed into the solvent tends to change in response to the characteristics of the solvent. The relative volatility of $C_1$ over $C_2$ in the presence of most lean oils is essentially the same as if the lean oil were absent, i.e., if the selectivity were slightly less than 5.0. There would accordingly be a potential benefit if a preferential physical solvent could be substituted for lean oil in the absorber plants used for processing natural gas where there might be a possibility of changing the

-13-

absorption behavior in the absorber column and thereby improving the economics.

The lean oils as used in the existing lean oil absorbers are essentially paraffinic base and form an essentially homogeneous mixture of paraffinic hydrocarbons which are of substantially higher molecular weight than the normal hydrocarbons present in a natural gas stream. Therefore they do not show any tendency to change the selectivity characteristics of the hydrocarbon components of the natural gases being treated.

The preferential physical solvents are defined for the purposes of this invention as having a minimum relative volatility of methane over ethane of at least 5.0 (thereby defining its improved selectivity toward ethane over methane) and in addition a solubility of at least 1.77 normal cubic meters of gaseous hydrocarbons per cubic meter of the solvent ($Nm^3/m^3$) (thereby defining its hydrocarbon loading capacity), or, alternatively, a preferential factor of at least 8.85. The preferential factor for physical solvent selection for the Mehra Process is defined as a product of relative volatility of methane over ethane multiplied by the solubility of ethane in physical solvents, specified as normal cubic meters of ethane per cubic meter of solvent. However, the ideal preferential physical solvent would have a selectivity toward ethane over methane of at least 10.0 and would simultaneously possess a hydrocarbon loading capacity of at least 21.2 $Nm^3/m^3$. This combination of minimum relative volatility and minimum solubility

0218359

-14-

enables solvent flow rate variations and operating pressure and temperature variations to be selectively utilized in the Mehra Process for flexibly producing liquid products having selected hydrocarbon compositions.

For example, the relative volatility for methane with respect to ethane in the presence of dimethyl ether of polyethylene glycol (DMPEG) is 6.4, indicating that it is more selective toward ethane than many other absorption liquids. As other examples, N-methyl pyrrolidone (NMP) and dimethyl formamide (DMF) have relative volatilities of methane/ethane of 5.3 and 8.5, respectively. However, the solubility of hydrocarbons in NMP is 0.2 $Nm^3/m^3$ and in DMF is 0.28 $Nm^3/m^3$; these values are low when compared to 7.0 $Nm^3/m^3$ for DMPEG. It is the combination of these factors that determines the effectiveness of physical solvents. In general, if a choice must be made, it is better to have a high preference factor than a high loading factor, although high levels of both factors are needed for really good performance in the Mehra Process.

Relative Volatilities of Ethane/Methane, Solubilities of Ethane in Solvent, and combined Preferential Factors are given in the following Table II for NMP, DMF, a common lean oil, the minimum solvent having desired requirements for Mehra Process applications, DMPEG, mesitylene, and the ideal solvent in the Mehra Process. These preferential factors are important because they inversely indicate the amount of solvent which is required, in terms of solvent flow rate, for a given recovery level of desirable
hydrocarbons. In other words, as 45 is much greater
than 1.1, the amount of NMP required is approximately
40 times the amount of DMPEG required for the same
performance.

TABLE II

Relative Volatilities of Ethane/Methane,
Solubilities of Ethane in Solvent, and
Preferential Factors Defining Preferential Physical Solvents
for Mehra Process Applications

| SOLVENT | NMP | DMF | LEAN OIL | MIN. | DMPEG | MESITYLENE | MIN. FOR IDEAL |
|---|---|---|---|---|---|---|---|
| Relative Volatility ($\alpha$) | 5.3 | 8.5 | 2.5-4.8 | 6.0 | 6.4 | 6.9 | 10 |
| Solubility, $Nm^3/m^3$, $\gamma$ | 0.2 | 0.28 | 1.4-7.0 | 7.0 | 7.0 | 29.7 | 22 |
| Preferential factor ($\alpha \times \gamma$) | 1.1 | 2.4 | 3.5-34 | 42 | 45 | 205 | 220 |

The process of this invention uses a preferential physical solvent for extracting ethane and heavier hydrocarbon components from a gas stream, such as a hydrocarbon gas stream, at any desired ethane recovery from 2% to 98% while recovering 99+% of propane and all heavier hydrocarbons. Using the same solvent, the process of this invention can achieve any desired propane recovery from 2% to 99+%, while recovering 99+% of butanes and all heavier hydrocarbons without recovering more than 2% of ethane.

This invention process produces a liquid hydrocarbon product having a composition which is selectively versatile rather than fixed, as in prior art processes. In consequence, the composition of this hydrocarbon product, as in the previously disclosed embodiments of the Mehra Process, can be readily adjusted in accordance with market conditions so that profitability of the absorption operation can be maximized at all times and on short notice. Such versatility is achieved by flexibility in operating additions and steps. Specifically, the operator must consider and selectively change or vary the flow rate of the preferential solvent with respect to the flow rate of the hydrocarbon gas stream. The flow rate may be varied within the range of 0.1 to 70 cubic meters of solvent per thousand normal cubic meters of hydrocarbon gas, such as natural gas. He must additionally selectively vary the temperature at the bottom of the ES column.

This process also effectively utilizes a preferential physical solvent in a new or existing absorber plant, a new or existing refrigeration absorption plant, or a new or existing extractive stripping plant. For this purpose, the solvent has a relative volatility of methane over ethane of at least 6.0, an ethane solubility of at least 7.0 $Nm^3/m^3$, and a preferential factor of at least 42 and is useful for replacing the absorber lean oil for which an existing plant was designed, thereby producing ethane and propane in hitherto unavailable quantities. It has also been discovered that, by restricting the preferential physical solvent to these higher values of relative volatility, solubility, and preferential factor, the effective selectivity of the preferential physical solvent for this invention is thereby enhanced.

It has been found that it is surprisingly feasible to recover additional $C_2$ and $C_3$ hydrocarbon products without significantly modifying the existing equipment because the selectivity of such preferential physical solvent permits such recoveries without simultaneously requiring processing of substantial amounts of undesirable components such as methane or ethane, depending upon the desired objectives of the recovery process.

With the use of a monocyclic aromatic physical solvent having $C_8-C_{10}$ hydrocarbons as a preferential physical solvent to extract a selected hydrocarbon component and heavier hydrocarbon components to a selected degree from a gaseous stream of $C_1$+ hydrocarbons, the process of this invention is as useful in a new plant for treating hydrocarbon gas streams as it is in rejuvenation of an existing plant and can facilitate reduced construction costs therefor because the extraction, stripping, and distillation columns can be reduced in size and the solvent pumps, heat exchangers, and the like can also be of smaller size in view of the smaller solvent inventory and lower reflux ratio that are needed.

It is important to note the difference between the processes of absorption and extraction. In "Mass Transfer Operations" by Robert E. Treybal, McGraw Hill Book Company, Second Edition, 1968, pages 220-226, absorption has been defined as an operation in which a gas mixture is contacted with a liquid for the purposes of preferentially dissolving one or more components of the gas and providing a solution of one or more of these components in the liquid. The solubility of a gas in an absorption medium is of primary importance. Since butanes and pentanes form an ideal solution with higher molecular weight paraffin oils, the solubility of the gas is the same in terms of mole fractions for all such solvents. These systems follow Raoult's and Henry's laws which define solubility as a function of partial pressures.

On page 395 of the above reference, Treybal defines the primary requirement of a satisfactory extractive distillation solvent as being its ability to be highly selective or possess the ability to alter the vapor-liquid equilibrium of the original mixture sufficiently to permit its easy separation, with, however, use of only small quantities of the solvent being desired.

In addition, John H. Perry in "Chemical Engineers' Handbook" Fourth Edition, McGraw-Hill Book Company, pages 13-46 and 13-47, defines extractive distillation as the addition of a separating agent, i.e., a solvent, to the column to alter favorably the relative volatilities of the feed components. However, the solvent must usually be added in fairly large amounts so that column diameters and heat loads are increased, and plate efficiencies may be lowered. It is further defined that the most important characteristics of the solvent are its selectivity and capacity. Selectivity is defined as the relative volatility of the feed components in the presence of the agent divided by the relative volatility of the feed components in the absence of the agent. The capacity of the solvent is defined as the liquid concentration of the feed components in the solvent in which the selectivity is measured.

In absorption, only solubility plays a role. In extraction, both solubility and selectivity play important roles. Solubility implies molecules entering the solvent surface, but selectivity involves molecules escaping from the solvent surface. Solubility is proportional to partial pressure, as stated by Henry's Law. Selectivity defines the relative volatility of one hydrocarbon, for example, to another hydrocarbon with respect to the same solvent, as $C_2$ to $C_1$ or $C_3$ to $C_1$ or $C_3$ to $C_2$.

The absorption principle leads to an alpha or relative volatility for methane with respect to ethane

0218359

-21-

of slightly less than 5 for almost all known absorption liquids. However, the relative volatility for methane with respect to ethane in the presence of dimethyl ether of polyethylene glycol (DMPEG) is 6.4, indicating that it is more selective toward ethane than many other absorption liquids.

The effect of substituting a preferential physical solvent for a lean oil in a new plant or in an existing plant is to "spread apart" the selectivities of the extracted hydrocarbons. Such "spreading" is indicated in the following Table III for ethane and propane with respect to methane, using the R values in "High $CO_2$-High $H_2S$ Removal with SELEXOL SOLVENT", by John R. Sweny, which was presented at the 59th Annual GPA Convention, March 17-19, 1980, Houston, Texas:

TABLE III

Comparative Selectivity Spreading for $C_1$, $C_2$, and $C_3$
Hydrocarbons in Two Solvents

| Solvents | $\alpha$ Values | | | Spread Between $C_3$ and $C_2$ | Spread Between $C_2$ and $C_1$ |
|---|---|---|---|---|---|
| | $C_3$ | $C_2$ | $C_1$ | | |
| Typical Lean Oil | 12.5-13.0 | 5.0 | 1.0 | 7.5-8.0 | 4.0 |
| Physical Solvent (e.g., DMPEG) | 15.3 | 6.4 | 1.0 | 8.9 | 5.4 |

0218359

-23-

The advantage imparted by such spreading apart of $\alpha$ values by a preferential physical solvent is that the selected solutes can be separated more easily (e.g., $C_1$ from $C_2+$ hydrocarbons) within the column-confined descending solvent droplets and ascending gases as equilibrium is reached by each hydrocarbon solute.

Selectively rejecting methane, methane plus ethane, methane plus ethane plus propane, or methane plus ethane plus propane plus butanes takes place within the ES column. Depending upon the liquid product specifications and the inlet hydrocarbon gas composition, the rejecting of undesirable hydrocarbons occurs in accordance with the temperature at the bottom of the ES column. Essentially, the stripping section of the ES column functions much like the demethanizing or stripping step of the extractive flashing version of the Mehra Process because in the stripping section, emphasis is placed on keeping undesired lighter hydrocarbons from coming down the column. In the extraction section, in contrast, emphasis is placed upon keeping the desired heavier components from continuing up the column and exiting as a part of the residue gas.

Thus, in a process for the removal of hydrocarbon gas liquids, comprising hydrocarbons heavier than methane from a hydrocarbon gas stream, wherein a need exists for recovering to any selected degree and at extremely high recoveries a selected hydrocarbon component and all heavier hydrocarbons within the group consisting of ethane, propane,

-24-

butane, pentane, and heavier hydrocarbons, without the need simultaneously to recover hydrocarbons lighter than the selected hydrocarbon component from the hydrocarbon gas stream,

This invention provides the improvement of selectively extracting the hydrocarbon gas liquids from the hydrocarbon gas stream with a preferential physical solvent which provides selective capability for recovery according to the selected degree of (a) ethane in amounts ranging from 2-98%, (b) propane in amounts ranging from 2-99%, (c) butane in amounts ranging from 2-100%, or (d) pentane and higher molecular weight hydrocarbons in amounts ranging up to 100% which comprises:

   A.  selectively extracting and stripping the hydrocarbon gas stream with the physical solvent to produce a residue hydrocarbon gas stream of pipeline specifications and a rich solvent stream containing ethane and heavier hydrocarbon components, the solvent being:

   1)  rich in $C_8$-$C_{10}$ aromatic compounds having methyl, ethyl, or propyl aliphatic groups and

   2)  selective for ethane and heavier hydrocarbon components of the gas stream such that: (a) the relative volatility of methane over ethane is at least 5.0 and the hydrocarbon loading capacity, defined as solubility of ethane in solvent, is

0218359

-25-

at least 1.77 normal cubic meters of ethane per cubic meter of solvent, or (b) the preferential factor, determined by the multiplication of relative volatility of methane over ethane by the solubility of ethane in solvent, in normal cubic meters of ethane per cubic meter of solvent, is at least 8.85; and

B.    distilling the rich solvent to produce the hydrocarbon gas liquids and the physical solvent.

The process of this invention thereby also produces a gas or liquid hydrocarbon product from a new plant or an existing absorption plant or from an existing refrigerated absorption plant which has a composition that is selective in accordance with the plant economics, is exceptionally superior with respect to minimum content of methane or methane and ethane, and is unusually valuable because of enhanced contents of economically desirable components such as ethane, as compared to products obtained with prior art absorber lean oil when utilized in the same equipment.

If the lean oil plant is of the absorption type, the recovery of propane can be significantly increased by using alkyl substituted monocyclic aromatic $C_8$-$C_{10}$ hydrocarbons as the solvent. Mesitylene, for example, gives excellent results, and a mixture of alkyl substituted monocyclic aromatic

0218359

-26-

$C_8$ hydrocarbons improves propane recovery even more, without any noteworthy change in piping or other equipment. However, propane yields can be further increased by recycling of streams of $C_1$-rich gases to the existing absorber, operating as an extractor with preferential solvents or mixtures thereof if such $C_1$-rich streams are available, as taught in U.S. Patent Nos. 4,421,535, 4,511,381, and 4,526,594. Other equipment changes that are known in the art can also be made and are likely to be economically worthwhile because the preferential physical solvent used in the process of this invention is a powerful tool that responds to available opportunities.

If the lean oil plant is of the extractive distillation or extractive stripping type, propane recoveries are even higher, for each respective solvent, than the propane recoveries in the absorption plant. Extractive distillation or stripping of the feed gas, as is known in the art, can be followed by a wide variety of distillation or stripping columns for solvent regeneration, product recovery, and the like. These units can affect yields of $C_3+$ hydrocarbon recovery, but in any such plant, it is generally true that the use of a preferential physical solvent, within the selectivity and volatility or preferential factor limits of the invention, gives surprisingly better results than lean oil performance.

The use of a preferential physical solvent in an existing absorber plant, so that the plant operates in extraction mode, causes the $C_2+$ hydrocarbon components to change their relative volatility relationships to each other as clearly as to methane. The attraction of ethane, for example, toward the preferential physical solvent is relatively so much greater than toward a lean oil that it may be thought of as tending to "crowd out" or displace the methane as the ethane becomes fully absorbed (i.e., to the capacity of the solvent) at the proper balance of gas and solvent flow rates.

However, at the same gas flow rate and at a lesser solvent flow rate, the propane may be thought of as tending to displace both the methane and the ethane as it becomes fully absorbed by the preferential physical solvent. Because of the change in absorptive behavior, an absorption operation becomes an extraction operation in the process of this invention.

The natural gas stream used as feedstock is selected from the group consisting of:

A.   natural gas at up to saturation with water;

B.   sweet natural gas;

C.   dry natural gas;

D.   sour natural gas which is pre-sweetened in gas phase with an aqueous amine solution and dried with an aqueous glycol solution; and

E.   sour natural gas.

The replacing column may be designed as an extraction column having a reboiler and a stripping section, whereby additional selectivity is provided to the improved process by carrying out the selectively extracting and stripping of the natural gas stream with the preferential physical solvent, thereby gaining an additional degree of freedom.

This additional degree of freedom is effectively utilized by appropriately selecting the reboiling temperature at the bottom of the column in order to produce the rich solvent stream consisting essentially of only economically desirable hydrocarbons out of the ethane and heavier hydrocarbon components, thereby rejecting economically undesirable hydrocarbon components.

It sometimes happens that lean oil absorber plants are under-designed with respect to a single piece of equipment or a single unit operation, such as, for example, the absorber column, as compared to the remainder of the equipment. If the plant had not been run at capacity because of a slack market or inadequate gas supplies, the potential bottleneck would not have been noticed or at least would have occasioned no difficulty.

It would therefore make good economic sense to replace the undersized equipment, such as the absorber column, with full-sized equipment. Indeed, the absorber column could appropriately be replaced with an extractive stripper column having a reboiler at its bottom. However, operating the process with a replaced extraction column (without a reboiler) or with an extractive stripping column while using a preferential physical solvent instead of the lean oil and at a flow rate of 0.1-70 $m^3$ of lean solvent per thousand normal cubic meters of the inlet gas provides the capability of considerably exceeding past performance of the lean oil plant.

Suitable preferential physical solvents include dialkyl ethers of polyalkylene glycol, N-methyl pyrrolidone, dimethyl formamide, propylene carbonate, sulfolane, glycol triacetate, and streams rich in $C_8$-$C_{10}$ aromatic compounds having methyl, ethyl, or propyl aliphatic groups and specifically constituting a sub-group consisting of mesitylene, n-propyl benzene, n-butyl benzene, o-xylene, m-xylene, p-xylene, and mixtures thereof, and aromatic streams rich in mixed xylenes, $C_9$ alkylaromatics, and other $C_8$-$C_{10}$ aromatics, rich being defined with respect to the solvent as more than 15% by weight of the aromatic compound.

These aromatic compounds boil in the range of 130-220°C and are stable at the process temperatures used in separating mixtures into useful fractions and/or components, such as in distillation, extractive stripping, and extractive distillation operations. Moreover, they are also hydrocarbons which can be left in the liquid products in trace amounts, without interfering with use of such products in gasoline, for example, so that purification of the liquid products is not needed.

If a non-aromatic compound is selected as the solvent, the stripping operation must be performed at a lower pressure in order to reduce the temperature sufficiently to avoid solvent decomposition.

0218359

-30-

If the hydrocarbon gas stream is sour, it is preferred that it be sweetened by contact with an acid-absorbing solvent, such as an amine, for example, before the extraction-stripping process of this invention is utilized. However, if an amine pretreating step is not suitable, the sour hydrocarbon gas stream can be treated according to the instant process. The acidic components are then maintained in liquid-phase or vapor-phase solution or contact, respectively, with the heavier hydrocarbon components until the solution or mixture, as a liquid, can be contacted by an acid-absorbing solvent. Because such post-absorption sweetening is done in liquid phase, the capital cost for equipment is relatively low.

If the inlet hydrocarbon gas stream is contaminated with an inert gas, such as nitrogen, helium, or argon, or mixtures thereof, the inert gases leave the process with the residue gas stream as a mixture of inert gas and methane.

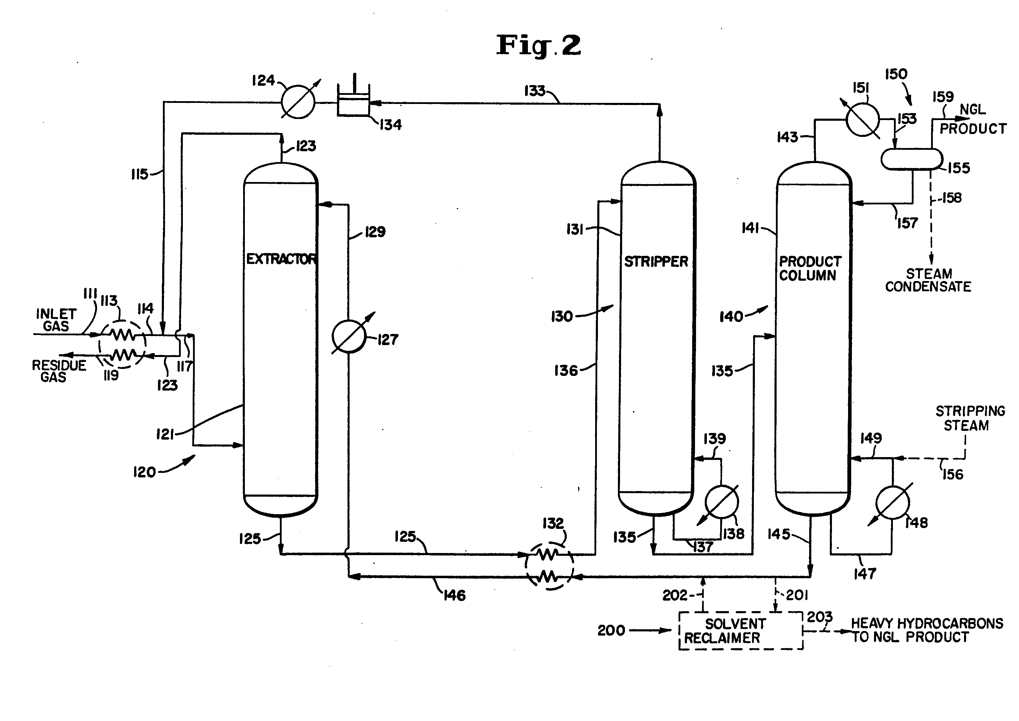

The invention may be more readily understood by referring to the drawings which diagrammatically illustrate preferred embodiments for treating hydrocarbon gases for removal of hydrocarbons heavier than methane from an inlet gas stream. In Figures 2-4, the arrangement of the apparatus is typically found in a conventional lean oil absorption plant.

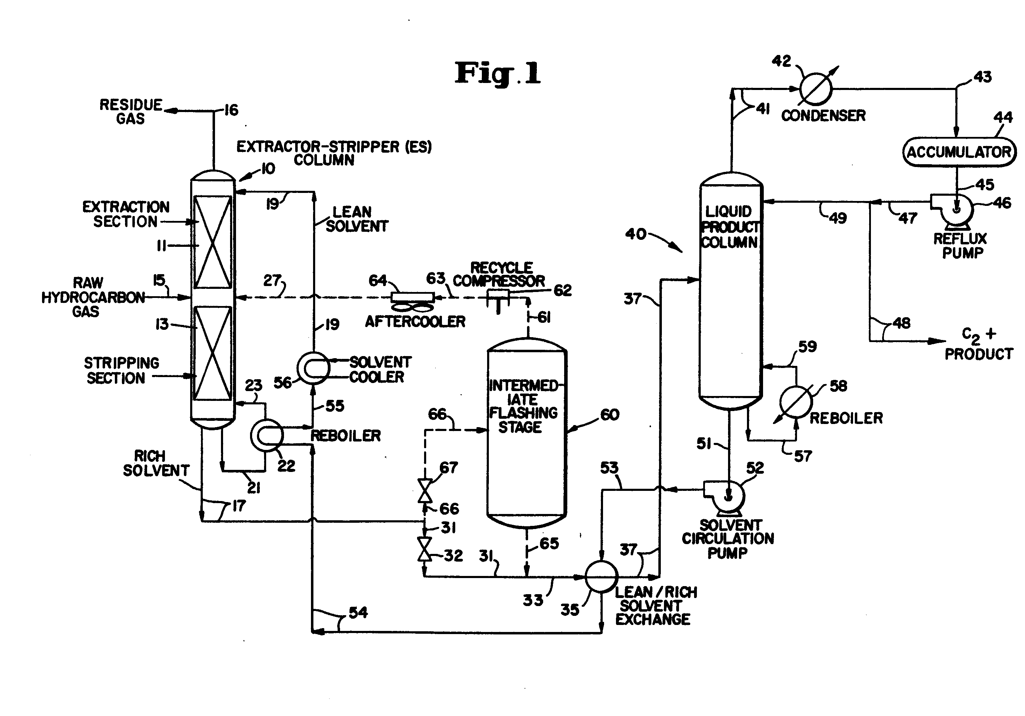

Figure 1 shows a schematic flow sheet for this invention process, having an Extractor-Stripper (ES) column and a distillation column, in which: (1) a preferential physical solvent extracts and strips selected $C_2+$ hydrocarbons from a raw hydrocarbon gas that is fed into the midsection of the ES column and (2) the rich solvent is regenerated in the distillation column which also produces a hydrocarbon gas liquids product as overhead.

Figure 2 is a schematic flow sheet for extraction of an inlet hydrocarbon gas stream and a recycle gas stream with a preferential physical solvent to produce a liquid product stream and a residue gas stream by using three treating units, two of which are a separate Extractor column and a separate Stripper column instead of a single ES column, the liquid and vapors flowing countercurrently between these columns. The two columns may operate at different pressure levels.

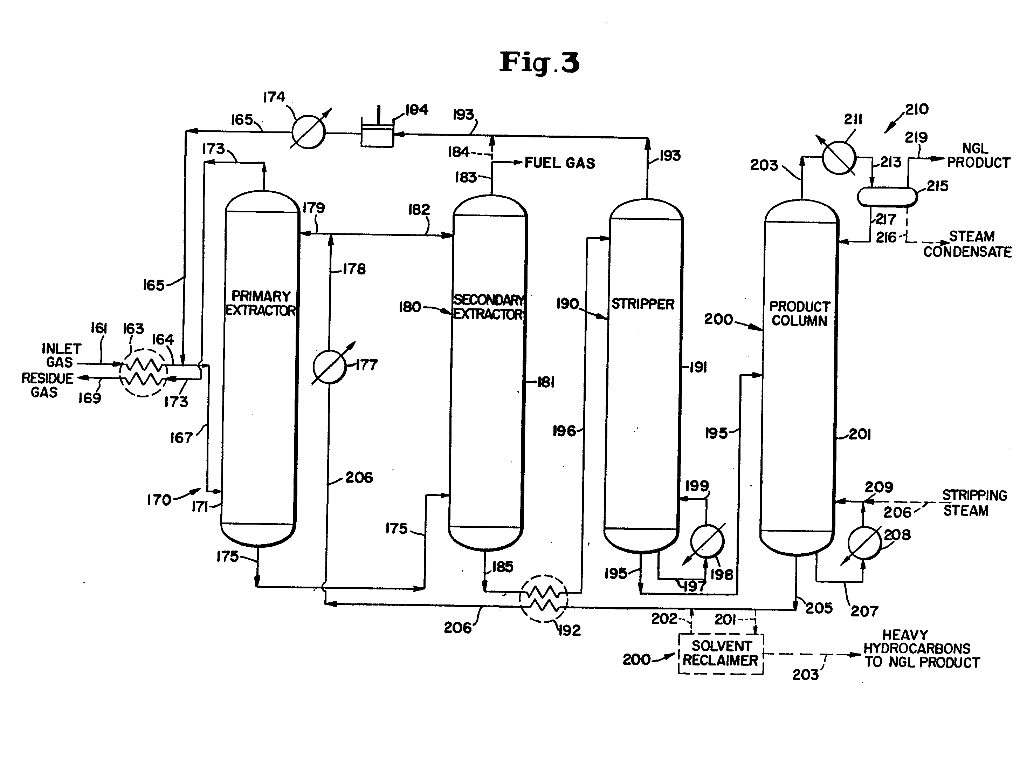

Figure 3 is a schematic flow sheet for extraction of an inlet gas stream and a recycle stream with a preferential physical solvent by using four treating units to produce a hydrocarbon gas liquids product stream and a residue gas stream having more exact specifications than the corresponding products of the process shown in Figure 1. The first three units act as a modified ES column.

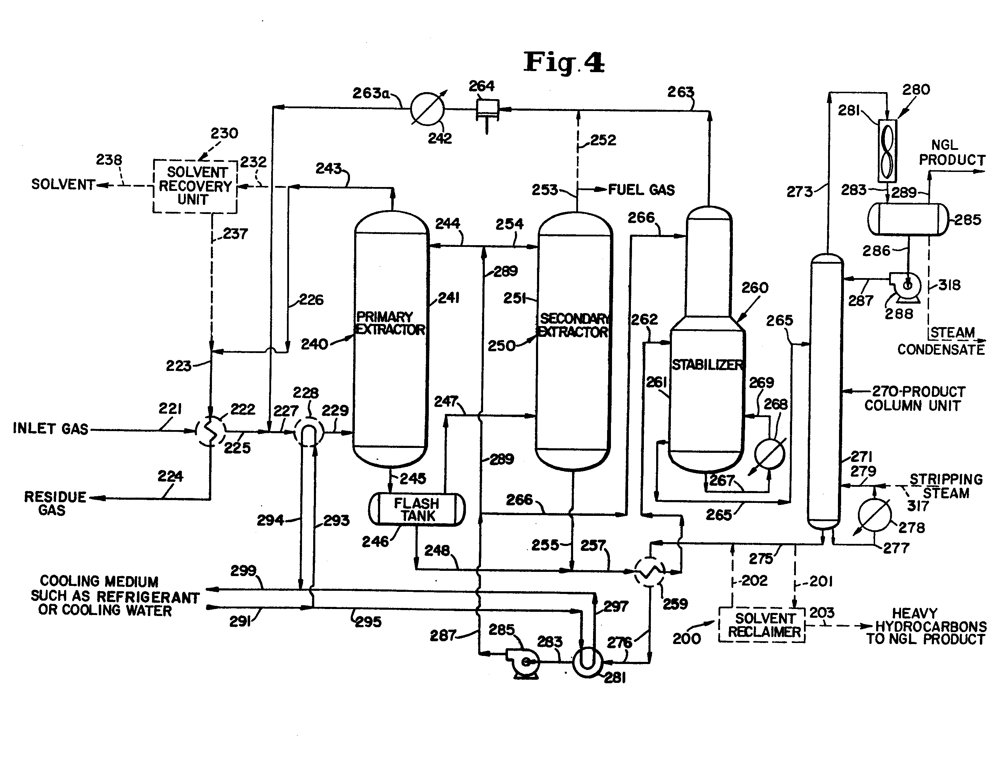

Figure 4 is a schematic flow sheet for extraction of an inlet natural gas stream and a recycle gas stream by using five treating units, wherein flashing to a lower pressure is used in at least one unit and reboiling is used in at least one unit, to produce a natural gas liquids product and a residue gas product having desired and exacting specifications with maximum thermal efficiency in the process.

-32-

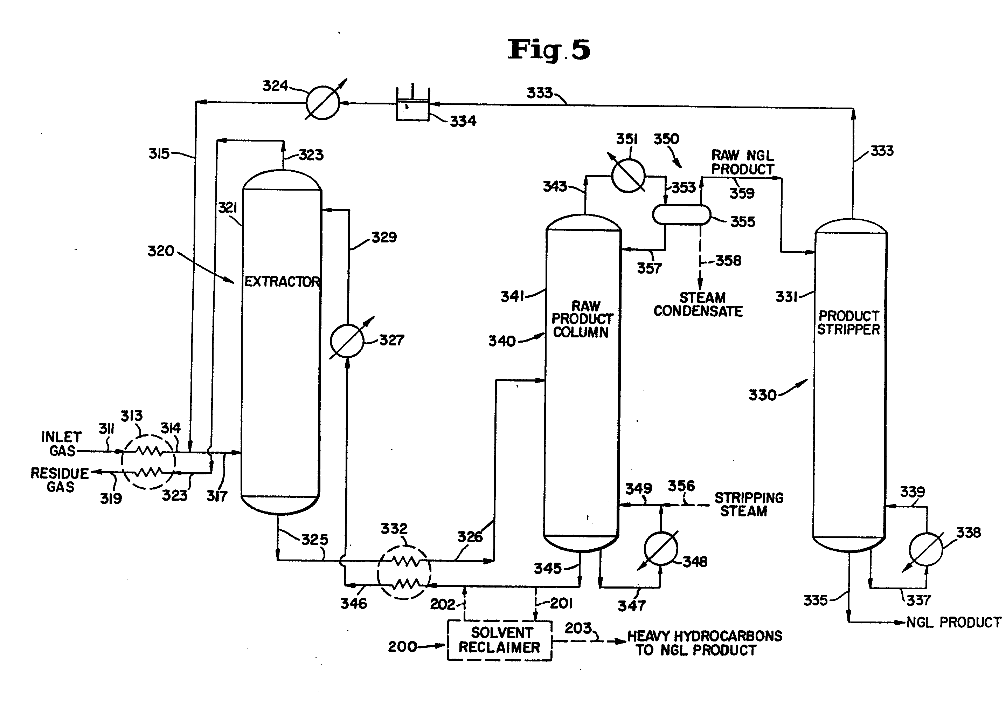

Figure 5 is a schematic flow sheet for extraction of an inlet gas stream and a recycle gas stream with a preferential physical solvent to produce a liquids product stream and a residue gas stream by using three treating units in which the product stripper column and the raw product column are reversed as compared to Figure 2.

With reference to the drawings, it should be understood that pipelines are in fact being designated when streams are identified hereinafter and that streams are intended, if not stated, when materials are mentioned. Moreover, flow-control valves, temperature-regulatory devices, pumps, and the like are to be understood as installed and operating in conventional relationship to the major items of equipment which are shown in the drawings and discussed hereinafter. The term, "absorber", is employed for an existing gas/solvent facility, but when it is utilized in the process of this invention with a preferential physical solvent, it is considered to be an "extractor".

As shown in the drawings, the improved Mehra Process of this invention, for selective extraction of hydrocarbon gas liquids from a gas stream, (such as the extraction of natural gas liquids from a stream of raw natural gas), comprises combined extraction and stripping to form a rich solvent and a residue gas, followed by distillation of the rich solvent to form the hydrocarbon gas liquids product and recycling of the lean solvent to the Extractor-Stripper column.

-33-

Extractor-Stripper (ES) column 10 comprises an extraction section 11 in its upper portion and a stripping section 13 in its lower portion. These sections 11, 13 may be mounted in separate columns. A stream of a raw hydrocarbon gas, such as a natural gas, in line 15 enters the midsection, between sections 11 and 13, of column 10 and passes countercurrently to a preferential physical solvent stream entering the top of extraction section 11 through line 19. Stripped vapors from stripping section 13 also rise with the entering hydrocarbon gas and meet the downwardly moving solvent. The solvent is lean with respect to hydrocarbons, "lean" being defined as any hydrocarbon content substantially below saturation. In an alternative embodiment, the raw hydrocarbon gas in line 15 is also combined with recycled gas entering the midsection of ES column 10 through line 27.

The solvent leaving extraction section 11, carrying absorbed $C_1$+ hydrocarbons, enters the top of stripping section 13 and passes countercurrently to vapors stripped from a pool of liquid in the bottom of column 10. This pool of liquid is kept at a selected boiling temperature and is boiled at a selected rate by heat which is produced in reboiler 22 as recirculated solvent in line 21 passes therethrough and returns through line 23 to the bottom of column 10. This heat may be economically provided by the lean solvent in line 54.

The rich solvent in line 17 from the bottom of column 10 passes through line 31 to valve 32 and then into line 33 before entering heat exchanger 35 for lean/rich solvent exchange. The warmed solvent in line 37 is next fed to the midsection of liquid product column 40. The overhead vapor stream leaves in line 41, passes through condenser 42, and discharges through line 43 into accumulator 44 from which liquid passes through line 45 to reflux pump 46. Pumped liquid in line 47 is partially returned to column 40 through line 49 as reflux, and the remaining amount is discharged as $C_2+$ hydrocarbon liquids product through line 48.

Bottoms from column 40 are discharged through line 51 to pump 52 as lean solvent for passage through line 53, exchanger 35, line 54, reboiler 22, line 55, and solvent cooler 56 before entering the top of ES column 10 through line 19. The liquid in the bottom of liquid product column 40 is maintained at a selected temperature by recirculation through line 57, reboiler 58, and line 59.

As an alternative embodiment, rich solvent in line 17 is sent through line 66 and valve 67 to intermediate flashing stage 60 which produces an overhead of flashed gases in line 61 which are compressed by recycle compressor 62 and passed through line 63 and aftercooler 64 before entering line 27 and feeding to the midsection of ES column 10. Alternatively, although not shown in Figure 1, this flashed gas stream may leave the process with residue gas stream 16. Bottoms from intermediate flashing stage 60 are discharged through line 65 to enter line 33, pass through exchanger 35, and enter the midsection of liquid product column 40 through line 37.

The process shown schematically in the flow sheet of Figure 2 comprises an extractor unit 120, a stripper unit 130, and a product unit 140. Inlet gas stream 111 enters an optional heat exchanger 113 and becomes cooled inlet gas stream 114 which combines with recycled gas stream 115 to produce combined gases 117 for use as feedstock to extractor unit 120. Depending upon operating temperatures in extractor column 121, cooling is supplied within optional heat exchanger 113 by overhead stream 123 which becomes heated residue gas stream 119, thereby recovering available cooling energy.

After entering column 121 of extractor unit 120 at a pressure which varies between 1,800-9,100 Kpa, feedstock 17 rises countercurrently to the downward flow of a lean preferential physical solvent stream entering the top of column 121 from line 129 after being cooled in cooler 127. The cooler 127 generally operates such that the temperature of lean solvent in stream 129 is -29°C or warmer. Overhead stream 123 leaves the top of column 121 for passage to heat exchanger 113. Bottoms in column 121 leave through line 125 to become feedstock for stripper unit 130.

Before entering column 131 of stripper unit 130, bottoms in line 125 pass through optional heat exchanger 132 to become heated bottoms in line 136. The pressure in extractor column 121 is at pressure in the order of 1,800-9,100 Kpa but the pressure in stripper column 131 may be at a lower pressure of the order of 800 to 9,100 Kpa, so that heated bottoms 136 flash when entering column 131. The flashed gases leave the top of column 131 through line 133, are compressed by compressor 134 and cooled by cooler 124, and pass through line 115 to join the inlet gas in line 114 and become combined feedstock in line 117 for column 121 at the inlet gas pressure.

Bottoms of column 131 are recycled through line 137 to reboiler 138 and line 139 for supplying heat to the contents of column 131. Since the vapors leaving as line 133 may contain some desirable components such as propane and ethane, the recycling of line 133 to the inlet to extractor unit 120 results in improved recoveries of the desirable components. Bottoms are discharged through line 135 to product column 141. Optionally the bottoms in line 135 may also be heated before entering the product column 141. Depending upon the relative operating pressure of product column 141, the bottoms in stream 135 may be pumped if the pressure in stripper column 131 is lower than the operating pressure of column 141.

Within column 141, bottoms 135 are further heated by passing through line 147, reboiler 148, and line 149. Overhead leaves through line 143 at the top of column 141, and bottoms leave through line 145 to pass through optional heat exchanger 132, line 146, cooler 127, and line 129.

Overhead stream 143 is cooled and condensed in condensing unit 150 and in particular in a partial or total condenser 151 and passes through line 153 to accumulator 155 from which a reflux stream enters column 141 through line 157, and a gas or liquid product stream leaves through line 159.

In summary, the purpose of extractor unit 120 is to selectively extract desirable components of inlet hydrocarbon gas stream 111 by effectively utilizing the selectivity of the preferential physical solvent in stream 129. The purpose of stripper unit 130 is to reject undesirable components of the inlet gas stream that may be present in the rich solvent streams 125 and 136 and prevent them from leaving with the solvent and the desirable components that are present in stream 135.

The operating pressure of stripper 131 can be the same as that of extractor 121, but it is generally lower than the operating pressure of extractor 121 in order to affect the separation of undesirable components. Reboiler 138 may obtain heat energy either from an external source or as waste heat from the lean solvent in stream 145. Stripper unit 130 may be referred to as a stabilizer, de-ethanizer, demethanizer, stripper, and the like but has the same meaning as described above.

The purpose of product column unit 140 is to separate desirable hydrocarbons from the preferential physical solvent as present in line 135 in order to produce a lean solvent stream 145 for recycling to extractor unit 120. The operating conditions of product column 141 are selected such that the operating temperature at its bottom is no greater than the boiling point of pure solvent. Furthermore, the operating pressure at the top of the product column is selected such that the reflux can be condensed with a conventional cooling source such as ambient air or cooling water. Refluxing column 141 is important in order to minimize losses of the solvent with the hydrocarbon liquid product in stream 159.

Depending upon the relative concentration of desirable components in inlet gas stream 111 and recovery of those components, the condenser 151 may be a partial condenser for generating sufficient reflux a total condenseror for condensing the entire product. Product column 141 may utilize heat from external sources through reboiler 148, or solvent can be stripped by steam injection through line 156 via line 149, and the steam condensate can be recovered from overhead accumulator 155 via line 158.

Referring to Figure 3, inlet gas 161 enters an optional heat exchanger 163 and becomes cooled gas 164 which is joined by an overhead recycle gas stream 165 from compressor 194 and aftercooler 174 to become combined gases 167 as feedstock for primary extractor column 171 of primary extractor unit 170. Depending upon the operating temperatures of extractor column 171, overhead gas stream 173 also passes through optional heat exchanger 163 to become heated gas stream 169 as residue gas for feeding to a pipeline.

Lean preferential physical solvent, after cooling in cooler 177, passes through line 178, and a major portion flows to the top of primary extractor column 171 through line 179 and thereafter flows downwardly, countercurrently to the gases entering from line 167. Bottoms from column 171 are discharged through line 175 and become feedstock for column 181 of secondary extractor unit 180.

-39-

The remaining portion of the lean solvent in line 178 flows through line 182 to the top of column 181, thereafter flowing downwardly and countercurrently to upwardly flowing gases from line 175. Overhead is discharged through line 183 for use as fuel gas. The gases in stream 183 may be recycled via line 184 to line 193, however, if so desired. Bottoms are discharged from column 181 through line 185 to enter optional heat exchanger 192.

The heated solvent in optional heat exchanger 192 flows through line 196 to enter near the top of column 191 of stripper unit 190, as feedstock therefor. Overhead from stripper column 191 leaves through line 193 to enter compressor 194. -Bottoms are heated by passage through line 197, reboiler 198, and recycle line 199. Bottoms are discharged through line 195 to become feedstock for column 201 of product unit 200.

Overhead is discharged from the top of product column 201 through line 203, and bottoms are discharged through line 205 to pass through optional cooler 192 and line 206 to cooler 177. Bottoms in column 191 are heated by passage through line 207, reboiler 208, and recycle line 209.

Overhead stream 203 is cooled and condensed in partial or total condenser 211 of condenser unit 210, and the condensate passes through line 213 to accumulator 215, from which liquid is refluxed through line 217 to the top of product column 201. A gas or liquid product stream is removed from accumulator 215 through line 219.

-40-

The purpose of primary extractor unit 170 in Figure 3 is similar to the function of extractor unit 120 of Figure 2. However, secondary extractor column 181 operates at an intermediate pressure between the operating pressures of primary extractor column 171 and stripper column 191. The vapor in secondary extractor column 181 is created by lowering the pressure of rich solvent stream 175. In reducing the pressure, the contained undesirable components of inlet gas stream 161 are preferentially separated from the physical solvent. Unfortunately, some of the desirable hydrocarbons also vaporize, even though most of them stay with the rich physical solvent that leaves in stream 185. Since the relative partial pressures of the desirable hydrocarbons are substantially higher in the vapors created by flashing in unit 180, it is significantly easier to further improve the recovery of desirable hydrocarbons by subjecting these vapors to additional contact with lean physical solvent from stream 182. Thus, the combined solvent from line 182 and line 179 flows out of secondary extractor column 181 through line 185.

The functions of stripper unit 190 and product unit 200 are almost identical to the operating requirements described for units 130 and 140 of Figure 2. Similar to line 156 in unit 140 of Figure 2, stripping steam in line 206 may be effectively utilized, if available, for stripping desirable hydrocarbons from the solvent in order to produce a lean preferential physical solvent in stream 205, whereas the steam condensate is recovered from the system via stream 216.

-41-

Referring to Figure 4, inlet gas in line 221 passes through an optional cross exchanger 222 to enter line 225 and be joined by a recycle stream in line 263a to become a combined gas stream in line 227. This stream passes through an optional inlet gas chiller 228 and then through line 229, as twice-cooled inlet gas at a temperature of not less than -29°C, to become feedstock for column 241 of primary extractor unit 240.

Overhead from extractor 241 leaves through line 243 and may be subjected to an optional solvent recovery unit 230 by passing through inlet line 232 and outlet line 237 to join line 223 and then to pass through heat exchanger 222 and leave the process through line 224.

Recovered solvent leaves unit 230 through line 238. The solvent recovery may consist of a cryogenic unit, molecular sieve or alumina beds, or activated carbon beds for the removal of contained solvent in residue gas stream 243. The solvent content of stream 243 is a function of molecular weight and vapor pressure of the solvent and the operating conditions of temperature and pressure in primary extraction column 241. Depending upon the economics of solvent recovery unit 230, residue stream 243 may by-pass unit 230 via line 226 to line 223 for heating via optional exchanger 222 to enter residue gas line 224. This solvent recovery unit may also be effectively employed on stream 123 of Figure 2 and stream 173 of Figure 3 in a similar manner.

It may also be economically preferable to utilize a heavy hydrocarbon oil for recovering solvent

-42-

vapor from the residue gas stream in lines 123, 173 and 243. Such a heavy hydrocarbon oil should have molecular weights in the order of 180 to 230 and should be fed to a small section of the primary extraction column above the point where the preferential physical solvent enters the column. The hydrocarbon oil, which now contains extracted solvent, never enters the extraction section and is removed from the primary extractor column. The removed hydrocarbon oil can be fractionated to recover solvent overhead for recycle to the preferential physical solvent loop, while the regenerated hydrocarbon oil is recycled back to the solvent recovery section.

The lean preferential physical solvent enters column 241 through line 244 and flows countercurrently to feedstock in stream 229. Bottoms leave column 241 in line 245 and are flashed from a pressure of 1,800 to 9,100 Kpa to a pressure of 800 to 4,200 Kpa in flash tank 246. Flashed gases leave through line 247 to enter column 251 of secondary extractor unit 250 as feedstock therefor. Bottoms leave tank 246 through line 248.

A lean solvent stream enters the top of column 231 through line 254 and passes downwardly and countercurrently to the upwardly moving stream of gases from line 247 within column 251. The overhead from column 251 leaves through line 253 as fuel gas or may be recycled to stream 263 via line 252. Bottoms from column 251 leave in line 255 and are joined by the solvent stream in line 248 to become a combined stream in line 257 which then passes through an optional cross exchanger 259.

-43-

The mixture of solvent and hydrocarbon components in line 262, after passing through cross exchanger 259, is fed to stabilizer column 261 of stabilizer unit 260, at the same time dropping from a pressure of about 800 to 4,200 Kpa to a pressure of about 700 to 2,900 Kpa within column 261. A portion of the cool lean solvent is fed to column 261 through line 266. Overhead from column 261 leaves through line 263, is compressed by compressor 264, cooled by an aftercooler 242, and passes through line 263a to join line 225 for recycling to column 241. Bottoms from column 261 are heated by passing through line 267, reboiler 268, and recycle line 269 to column 261. Discharged bottoms leave column 261 through line 265 and become feedstock for product column unit 270.

An overhead stream from column 271 leaves through line 273, and bottoms leave through line 275 and then pass through optional cross exchanger 259, line 276, chiller 281, line 283, pump 285, and line 287 before splitting into a portion going through line 266 to the top of column 261 and the remaining portion passing through line 289 to split into two portions in lines 244 and 254 for respective columns 241 and 251.

The bottoms of column 271 is reboiled via line 277, reboiler 278 and recycle line 279 to column 271. Optionally the physical solvent is stripped with stripping steam via line 317 while the steam condensate is recovered via line 318.

The overhead in line 273 is condensed in still reflux condenser 281 and passes through line 283 to reflux accumulator 285, wherein reflux is separated and

0218359

-44-

passes through line 286 to pump 288 and line 287 to column 271 as reflux at the top thereof. Hydrocarbon products are removed from accumulator 185 through line 289.

Cooling medium such as cooling water or refrigerant enters the process through line 291 and is split into a portion going to optional heat exchanger 228 through line 293 and a portion moving through line 295 to heat exchanger 281. The warm cooling medium leaves through line 297 to be joined by the other portion in line 294 and becomes the combined stream in line 299. Typically, the temperature of the lean solvent in line 283 is warmer than -29°C.

In Figure 4, stabilizer unit 260 is slightly different from stripper unit 130 of Figure 2 and stripper unit 190 of Figure 3, in the sense that a small portion of the lean solvent is added as reflux to the top of column 261 via line 266. This affects preferential recovery of desirable hydrocarbons, thereby taking advantage of improved partial pressures of desirable hydrocarbons in column 261. Figure 4 is also different from Figure 2 in another aspect, wherein rich solvent stream 245 is flashed in a separate unit 246, but only vapors are subjected to solvent extraction in secondary extractor unit 250.

The process shown schematically in the flow sheet of Figure 5 comprises an extractor unit 320, a raw product unit 340, and a product stripper unit 330. Inlet gas stream 311 enters an optional heat exchanger 313 and becomes cooled inlet gas stream 314 which combines with recycled gas stream 315 to produce combined gases 317 for use as feedstock to extractor unit 320. Depending upon operating temperatures in the extractor column, cooling is supplied within the optional heat exchanger 313 by overhead stream 323 which becomes heated residue gas stream 319, thereby recovering available cooling energy.

After entering column 321 of extractor unit 320 at a pressure which varies between 1,800 and 9,100 Kpa, feedstock 317 rises countercurrently to the downward flow of a lean preferential physical solvent stream entering the top of column 321 from line 329 after being cooled in cooler 327. Cooler 327 generally operates such that the temperature of lean solvent in stream 329 is -29°C or warmer. Overhead stream 323 leaves the top of column 321 for passage to heat exchanger 313. Bottoms in column 321 leave through line 325 to become feedstock for raw product unit 340.

Before entering column 341 of raw product unit 340, bottoms in line 325 passes through optional heat exchanger 332 to become heated bottoms in line 326. The pressure in extractor column 321 is at a pressure of the order of 1,800-9,100 Kpa, but the pressure in raw product column 341 may be at a lower pressure of the order of 800 to 9,100 Kpa, so that heated bottoms 326 flash when entering column 341.

Bottoms in column 341 are further heated by passing through line 347, reboiler 348, and line 349. Overhead leaves through line 343 at the top of column 341, and bottoms leave through line 345 to pass through optional heat exchanger 332 and enter cooler 327 through line 346.

Overhead stream 343 is cooled and condensed in condensing unit 350 and in particular in a partial or total condenser 351 and then passes through line 353 to accumulator 355 from which a reflux stream enters column 341 through line 357, and a gas or liquid raw product stream enters product stripper column 331 through line 359.

The flashed gases leave the top of column 331 through line 333, are compressed by compressor 334 and cooled by cooler 324, and pass through line 315 to join the inlet gas in line 314 and become combined feedstock in line 317 for column 321 at the inlet gas pressure. Bottoms of column 331 are recycled through line 337 to reboiler 338 and line 339 for supplying heat to the contents of column 331. Since the vapors leaving in line 333 may contain some desirable components such as propane and ethane, the recycling of line 333 to the inlet to extractor unit 320 results in improved recoveries of the desirable components.

There are some preferential physical solvents such as mesitylene, mixed xylenes, reformates, and the like which may have lower average molecular weight and more relative volatility when compared to some of the absorption lean oils that are used in existing absorption type plants. If some of these gas streams, used as feedstock for the process, contain minor amounts of heavier hydrocarbons in comparison to the proposed preferential physical solvent, these heavier hydrocarbons will tend to build up in the circulating solvent and thus retard the effectiveness of the physical solvent because the final product column would

be unable to separate the heavier hydrocarbons from the physical solvent. Therefore, as part of this invention, utilization of solvent reclaimer unit 200 that is shown in Figures 2, 3, 4, and 5, for example, is equally applicable to the flow schematic of Figure 1 and can effectively prevent the build up of hydrocarbons in the solvent loop. In order to minimize their impact on solvent selectivity, the hydrocarbon content should be limited to less than 10% by volume and preferably to less than 5%. The volume of . slipstream 201 is a function of the proportionate quantity of heavier hydrocarbons in the inlet gas stream and is also simultaneously a function of the quantity of hydrocarbons in the regenerated solvent stream leaving the bottom of the fractionator or regenerator.

As shown in Figures 2, 3, and 4, a slip stream of hot lean solvent is sent via line 201 to a solvent reclaimer unit 200 which is operating at low pressures of up to 15 Kpa but is preferably operating at 140 Kpa or higher and utilizing external heat to boil off the physical solvent from the mixture and return it to the solvent loop via line 202. The separated hydrocarbons are discharged via line 203.

Alternatively, the solvent reclaimer section may consist of a fractionating still, if economical to do so as determined by the installation cost versus the reduction in solvent loss along with the heavier hydrocarbons, in order to fractionate the physical solvent in stream 201 from the heavier hydrocarbons. The fractionating still can be designed to minimize the

-48-

loss of solvent with the heavier hydrocarbons that may end up in the NGL product.

It should be recognized that because the molecular weights of heavier components are generally unknown and cannot be compared to those of the preferential physical solvent of this invention, the separation is somewhat difficult in the sense that it may require a large number of theoretical stages, of course balanced by the appropriate amount of reflux. In the final analysis, economics of the process, when weighed against the cost of solvent loss in the product, must be considered.

As an additional preferred alternative to the removal of such heavier hydrocarbon components from the physical solvent, an analysis of the inlet gases in streams 15, 111, 161, 221, or 311 may be made to determine if such components can be separated from the vapor stream before contacting with solvents in primary extraction units 10, 120, 170, 240 or 320. If so, it may be economically desirable to remove such components by installing a separating vessel after coolers, such as 113, 163, 228, or 313, thereby effectively separating condensed liquids. The vapor portion flows from this separator towards extraction units 10, 120, 170, 240, or 320 while the separated liquid combines with the hydrocarbon product in streams 48, 159, 219, 289, or 335. If the separated liquid contains some undesirable components such as methane and ethane, the separated liquid may be treated in a relatively small stripping column, thereby stripping methane and ethane along with minor amounts of $C_3$ and heavier, as long

-49-

as the stripped vapors do not contain hydrocarbons heavier than the physical solvent. The stripped vapors can be conveniently treated in stripper units 60, 130, 180, 190, 260, or 330.

EXAMPLE 1

A natural gas processing plant, having the equipment shown in Figure 4 and using the process described hereinbefore, is operated with lean absorption oil which has a molecular weight of 180, normal boiling point of 238°C and a gravity of 40° API. The inlet gas stream in line 221 flows at 7.39 million normal cubic meters per day or 308 kNm$^3$/hr and is at 29°C. It is at 16°C in line 229 as it enters column 241 which operates at 6,300 Kpa. Column 251 operates at 2,800 Kpa. Column 261 operates at 1,900 Kpa. Column 271 operates at 800 Kpa.

Lean absorption oil in line 287 is at 10°C and is split in three ways to provide 236 cubic meters per hour (m$^3$/hr) in line 244, 15 m$^3$/hr in line 254, and 42 m$^3$/hr in line 266, totalling 293 m$^3$/hr, to respective columns 241, 251, and 261.

The inlet gas stream contains 64 kg-mole/hr of nitrogen and 120 kg-mole/hr of $CO_2$. The compositions of the inlet gas stream in line 221, the residue gas stream in line 243, the fuel gas stream in line 253, and the NGL product stream in line 289 are given in Table IV.

TABLE IV

MATERIAL BALANCE

| Components, in kg-mole/hr | Stream in Line No. | | | |
|---|---|---|---|---|
| | 221 | 243 | 253 | 289 |
| Methane | 11,493 | 11,136 | 357 | 0 |
| Ethane | 1,280 | 1,214 | 62 | 5 |
| Propane | 497 | 297 | 16 | 184 |
| $C_4^+$ Hydrocarbons | 291 | 12 | 0.5 | 279 |

EXAMPLE 2

The hydrocarbons recovery plant shown in Figure 4 and utilized for Example 1 is economically rejuvenated by replacing all of its lean absorption oil with one of the preferential physical solvents of this invention, namely, mesitylene. The temperature of stream 277 is 10°C. The flow rates of streams 244, 254, and 266 are, respectively, 236, 15, and 42 cubic meters per hour ($m^3$/hr), totalling 293 $m^3$/hr.

The flow rate of inlet gas line 221 is 308 thousand normal cubic meters per hour (kNm$^3$/hr), having the same composition shown in Table IV. The temperature in line 229 is 16°C, and the pressures in columns 241, 251, 261, and 271 are, respectively, 6,300, 2,800, 1,900, and 800 Kpa.

The compositions of the inlet gas stream in line 221, the residue gas stream in line 243, the fuel gas stream in line 253, and the NGL product stream in line 289 are summarized in Table V.

-51-

### TABLE V

### MATERIAL BALANCE

| Components, in kg-mole/hr | Stream in Line No. | | | |
|---|---|---|---|---|
| | 221 | 243 | 253 | 289 |
| Methane | 11,493 | 11,077 | 416 | 0 |
| Ethane | 1,280 | 1,199 | 78 | 4 |
| Propane | 497 | 184 | 14 | 299 |
| $C_4^+$ Hydrocarbons | 291 | 7 | 1 | 283 |

Thus, by simply substituting the absorption oil of Example I with a preferential physical solvent of this invention process, e.g., mesitylene, while operating the plant at the same conditions, i.e., with no changes in physical equipment, propane recovery improved from 37.1% to a surprising level of 60.2%.

### EXAMPLE 3

The hydrocarbons recovery plant shown in Figure 4 and utilized for Example 1 is economically rejuvenated by replacing all of its lean oil with one of the other preferential physical solvents of this invention, namely, mixed xylenes. The temperature of stream 277 is 10°C. The flow rates of streams 244, 254, and 266 are, respectively, 258, 16, and 47 cubic meters per hour ($m^3$/hr), totalling 321 $m^3$/hr.

The flow rate of inlet gas line 121 is 308 normal meters per hour ($Nm^3$/hr), having the same composition shown in Tables IV and V. The temperature

0218359

-52-

in line 129 is 16°C, and the pressures in columns 241, 251, 261, and 271 are, respectively, 6,300, 2,800, 2,000, and 800 Kpa.

The compositions of the inlet gas stream in line 121, the residue gas stream in line 143, the fuel gas stream in line 153, and the NGL product stream in line 189 are summarized in Table VI.

TABLE VI

MATERIAL BALANCE

| Components, in kg-mole/hr | Stream in Line No. | | | |
|---|---|---|---|---|
| | 221 | 243 | 253 | 289 |
| Methane | 11,493 | 10,992 | 501 | 0 |
| Ethane | 1,280 | 1,179 | 97 | 4 |
| Propane | 497 | 138 | 16 | 342 |
| $C_4^+$ Hydrocarbons | 291 | 1 | 0 | 290 |

In this example, the propane recovery is surprisingly improved by 14.6% over the use of mesitylene in Example 2 and also to a level of 68.9% when compared to a level of 37.1% in Example 1, when all other operating conditions are identical, by replacing the absorber oil with mixed xylenes as the preferential physical solvent and by increasing the solvent flowrate by only 9.2%. The example further illustrates that a mixed xylenes stream is relatively more selective towards propane than mesitylene, both being preferential physical solvents for this invention process. With an increase of 9.2% solvent flowrate, those skilled in the art of treating natural gas with absorption oil while minimizing ethane recovery would have anticipated an improvement of only about 9.2% in propane recovery, but it was surprisingly 14.6% better, because preferential physical solvents combine their selectivity characteristics with their solubility characteristics. This behavior is contrary to the absorption principle characterizing lean oils, in which solubility is the only parameter.

It should be understood from the preceding three examples and the preceding description that the process of this invention can economically rejuvenate an old absorber plant without modification of the apparatus and simply by substitution of one solvent for another. However, it is realistic to assume that pumps will be found to need new seals, trays in the absorber column will be found to be outmoded by newer designs, or one unit operation will have been found by experience to have been a bottleneck or will be ascertained by computer simulation to be inefficient with the new solvent. In such situations, a reasonable amount of plant retrofit should be expected, reasonable being interpreted as within the limitations of economic practicality, maximization of efficiency and profits, and the like.

Such retrofit can even include replacement of an entire column if such economic criteria are met, as in an absorption process for the removal of $C_2+$ hydrocarbons from a natural gas stream by absorbing the $C_2+$ hydrocarbons with a lean oil to produce a residue gas stream of pipeline quality and a rich solvent from

which the $C_2+$ hydrocarbons are recovered and wherein a need exists for recovering at maximum recoveries a selected hydrocarbon component and heavier hydrocarbons within the group consisting of ethane, propane, butane, and pentane without the need simultaneously to recover hydrocarbons lighter than the selected hydrocarbon component from the natural gas stream, the selected component being ethane, propane, or butane, and for recovering to a selected degree the hydrocarbon lighter than the selected hydrocarbon component when the lighter hydrocarbon is ethane or propane.

In such a situation, the process of this invention improves the hydrocarbon recovery process by substituting for the lean oil an extractant which is a preferential physical solvent and is selective for ethane and heavier hydrocarbon components of the inlet gas stream such that the relative volatility of methane over ethane is at least 6.0 and the solubility of ethane in the solvent is at least 7.0 normal cubic meters of ethane per cubic meter of the extractant or the preferential factor is at least 42.

It should also be understood that the process of this invention lends itself to the use of waste heat recovery, quite contrary to the other state-of-the art processes that are capable of recovering ethane and propane under high recovery levels. Similarly, the opportunity for integrating heat exchangers is extensive. Even though Figure 1 indicates units 13, 32, 27, 38, 48, and 51, Figure 2 shows units 63, 77, 92, 98, 108, and 111, and Figure 3 contains units 122, 128, 172, 159, 168, 215, and 181, any combination of

0218359

-55-

waste heat exchange, rich/lean solvent heat exchange, steam stripping in lieu of the product column reboiler, and additional side reboiling for columns 21, 31, 41, 71, 91, 101, 141, 151, 161, and 171 are to be understood as part of this invention process with the objective of maximizing the recovery of hydrocarbons under given economic criteria.

WHAT IS CLAIMED IS:

1. In a process for the removal of hydrocarbon gas liquids comprising hydrocarbons heavier than methane from a hydrocarbon gas stream,

wherein a need exists for recovering to any selected degree and at extremely high recoveries a selected hydrocarbon component and heavier hydrocarbons within the group consisting of ethane, propane, butane, and pentane, without the need simultaneously to recover hydrocarbons lighter than said selected hydrocarbon component from said hydrocarbon gas stream,

the improvement which provides the capability of selectively extracting said hydrocarbon gas liquids from said hydrocarbon gas stream with a stream of a preferential physical solvent according to said selected degree of (a) specification amounts of methane, (b) ethane in amounts ranging from 2-98% (c) propane in amounts ranging from 2-99%, (d) butane in amounts ranging from 2-100%, and (e) pentanes and higher molecular weight hydrocarbons in amounts ranging up to 100%, said improvement being characterized by:

A. contacting said hydrocarbon gas stream with said physical solvent stream, at selected solvent flow rates within the range of 0.1-70 cubic meters of solvent per thousand normal cubic meters of hydrocarbon gas, to produce a residue hydrocarbon gas stream of pipeline specifications and a downwardly moving solvent stream enriched in economically desirable hydrocarbon components, said solvent being selective for ethane and heavier hydrocarbon components of the gas stream such that:

1) the relative volatility of methane over ethane is at least 5.0 and the hydrocarbon loading capacity, defined as solubility of ethane in said solvent, is at least 1.77 normal cubic meters of ethane per cubic meter of solvent, or

2) its preferential factor, determined by the multiplication of said relative volatility by said solubility, is at least 8.85;

B. stripping said downwardly moving solvent stream to produce a stream of rejected hydrocarbon components and a rich solvent stream containing said desirable hydrocarbon components; and

C. distilling said rich solvent stream to produce said hydrocarbon gas liquids and said physical solvent stream for recycling to said Step A.

2. The improved process according to claim 1, characterized in that said hydrocarbon gas stream is selected from the group consisting of:

A. natural gas at up to saturation with water;

B. sweet natural gas;

0218359

-58-

C.  dry natural gas;

D.  sour natural gas which is pre-sweetened in gas phase with an aqueous amine solution and dried with an aqueous glycol solution; and

E.  sour natural gas.

3.  The improved process according to claim 2, characterized in that said preferential physical solvent is selected from the group consisting of dialkyl ethers of polyalkylene glycol, N-methyl pyrrolidone, dimethyl formamide, propylene carbonate, sulfolane, glycol triacetate, and streams rich in $C_8$-$C_{10}$ aromatic compounds having methyl, ethyl, or propyl aliphatic groups and specifically constituting a sub-group consisting of mesitylene, n-propyl benzene, n-butyl benzene, o-xylene, m-xylene, p-xylene, and mixtures thereof, and aromatic streams rich in mixed xylenes and other $C_8$-$C_{10}$ alkyl aromatics.

4.  The improved process according to claim 3, characterized in that:

A.  additional selectivity is provided by using a reboiler and a stripping section in an extraction column for producing:

1)  said upwardly moving stream of rejected hydrocarbon components and

2)  said rich solvent stream consisting essentially of only said desirable hydrocarbon components;

B.   said upwardly moving stream of rejected hydrocarbons is stripped with said downwardly moving enriched stream of preferential physical solvent, thereby gaining an additional degree of freedom which is effectively utilized by appropriately selecting the reboiling temperature at the bottom of said column in order to produce said rich solvent stream and said stream of rejected hydrocarbon components;

C.   some desirable hydrocarbons in said stream of rejected hydrocarbon components are preferentially recovered by mass transfer principles by transfer to said downwardly flowing physical solvent stream, whereby a stream of undesirable hydrocarbon components continues to move upwardly;

C.   said stream of undesirable hydrocarbon components is mixed with the incoming hydrocarbon gas stream to form a gaseous mixture which flows upwardly in an extraction section of said column, whereby said lean solvent preferentially recovers any of said desirable hydrocarbon components contained in said mixture and produces said residue gas stream and said enriched solvent stream.

5.   The improved process according to claim 4, characterized in that said rich solvent is let down

-60-

in pressure to a pressure level that is consistent with the operation of a distillation column for conducting said distilling of said step B of claim 1, thereby producing a stream of flashed gases which is recycled to said extraction section for joining said incoming hydrocarbon gas stream.

6.    The improved process according to claim 5, characterized in that:

A.    said rich solvent is heated before entering said distillation column in order to lower the reboiler heat load on said distillation column;

B.    said distillation column is a fractionation-type column which separates recovered hydrocarbon components from said rich solvent stream;

C.    said recovered hydrocarbon components leave the top of said distillation column as column overhead and are condensed to form said hydrocarbon liquids product; and

D.    a stream of lean solvent leaves the bottom of said distillation column, the temperature at said bottom being selected to ensure the recovery of all said desirable hydrocarbon components and being no higher than the boiling point of said physical solvent at said operating pressure.

7.    The improved process according to claim 6, characterized in that said column overhead is refluxed with a portion of said hydrocarbon liquids product in order to minimize loss of said physical solvent contained in said hydrocarbon gas liquids product.

8. The improved process according to claim 3, characterized in that said lean physical solvent stream is cooled before recycling to said contacting step as said preferential physical solvent.

Fig.1

Fig.2

# Fig.3

Fig.4

# Fig.5

European Patent Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | GB-A-2 142 041 (EL PASO HYDROCARBONS CO.) * Claims * | 1-3 | C 07 C 7/11 C 10 G 5/04 |
| | --- | | |
| A | US-A-2 630 403 (A.J. MILLER) | | |
| | ----- | | |

|  |
|---|
| TECHNICAL FIELDS SEARCHED (Int. Cl.4) |
| C 07 C 7/00 C 10 G 5/00 |

The present search report has been drawn up for all claims

| Place of search | Date of completion of the search | Examiner |
|---|---|---|
| THE HAGUE | 22-10-1986 | VAN GEYT J.J.A. |

EPO Form 1503 03 82